# EUROPÄISCHE PATENTSCHRIFT

(11) **EP 1 140 227 B1**
(45) Veröffentlichungstag und Bekanntmachung des Hinweises auf die Patenterteilung: **01.10.2003**
(21) Anmeldenummer: 99958443.6
(22) Anmeldetag: 20.12.1999
(51) Int. Cl.: A61L 15/42, A61L 15/52

(54) **INFEKTIONSSCHUTZ-TAMPON**
TAMPON WITH INFECTION-PROTECTION
TAMPON DE PROTECTION CONTRE L'INFECTION

(30) Priorität: 21.12.1998 CH 252498
(43) Veröffentlichungstag der Anmeldung: 10.10.2001
(73) Patentinhaber: IVF Hartmann AG, 8212 Neuhausen am Rheinfall (CH)
(72) Erfinder: MÄHR, Rodolfo, CH-8207 Schaffhausen (CH); MÄHR, Andreas, CH-8207 Schaffhausen (CH); ENDERLI, Heidi, CH-8048 Zürich (CH)
(74) Vertreter: Blum, Rudolf Emil Ernst
(86) Internationale Anmeldenummer: IB9902025
(87) Internationale Veröffentlichungsnummer: WO00037118

(56) Entgegenhaltungen:
- EP-A- 0 599 307
- AT-U- 654
- DE-A- 1 541 275
- US-A- 3 902 493
- US-A- 5 009 890
- US-A- 5 607 754
- US-A- 5 641 503
- ALTMAN P M: "AUSTRALIAN TEA TREE OIL-A NATURAL ANTISEPTIC" AUFBEREITUNGS TECHNIK, Bd. 3, Nr. 4, Oktober 1989 (1989-10), Seite 247/248 XP000783533 ISSN: 1443-9302

## Beschreibung

### Hintergrund

Die vorliegende Erfindung betrifft einen Tampon, der insbesondere für die Benutzung im Wasser, d.h. beim Baden oder Schwimmen, geeignet ist und die dabei existierende Infektionsgefahr vermindert oder sogar ganz beseitigt.

### Stand der Technik

Das Tränken und Imprägnieren von Tampons mit flüssigen Paraffinen und anderen Stoffen zur Verbesserung der Gleitfähigkeit des Tampons und damit zur Verbesserung dessen Einführbarkeit oder zur Verhinderung des Eindringens von Wasser in den Scheidengang beim Baden sind bekannt (siehe z.B. AT 000 654 U1)

Es ist aus DE-PS 19 25 086, DE-OS 20 24 930 und DE-OS 15 41 275 auch bereits bekannt, Tampons z.B. mit mineralischem Öl als Träger eines Medikaments, resp. einer wässrigen biologischen Heillösung, zu tränken, um das Medikament in genau vorgegebener Menge und Konzentration ins Innere des Scheidenganges einzubringen. US 5,641,503 offenbart einen Fettsäureester enthaltenden Tampon, der die Toxin-Produktion von Staphylococcus aureus verhindert.

In US 3 902 493 wird ein Tampon beschrieben, der einen Schaumkörper mit Hülle umfasst. Die Hülle kann mit einem therapeutischen Mittel und einem bindenden Schmiermittel imprägniert sein.

Es ist ferner bekannt, dass das Eindringen von Wasser in den Scheidengang Ursache für Infektionen sein kann. Ziel der vorliegenden Erfindung war es deshalb nicht nur das Eindringen von Wasser zu vermindern sondern gleichzeitig die Entstehung von Infektionsherden durch den nie vollständig vermeidbaren Kontakt mit dem Wasser zu verhindern oder zumindest stark zu vermindern.

### Darstellung der Erfindung

Dieses Ziel wurde erreicht durch das Bereitstellen eines speziellen, mit Imprägniermittel versehenen Tampons mit einem saugfähigen Tampon-Körper und einem einseitig aus dem Tampon-Körper austretenden Haltemittel zur Entfernung des Tampons aus der Scheide, der dadurch gekennzeichnet ist, dass das Imprägniermittel einen physiologisch unbedenklichen hydrophoben Träger und ein im wesentlichen ebenfalls hydrophobes und physiologisch unbedenkliches Desinfektionsmittel enthält. Das hydrophobe Desinfektionsmittel besteht aus Substanzen mit infektionshindernder oder zumindest infektionshemmender Wirkung. Diese Substanzen entfalten vorzugsweise antimikrobielle und/oder antibakterielle und/oder mycostatische und/oder bakteriostatische Wirkung.

Das hydrophobe Desinfektionsmittel umfasst vorzugsweise ein pflanzliches Öl mit der oder den entsprechenden Wirkungen oder eine Mischung von solchen pflanzlichen Ölen. Speziell bevorzugt ist, dass das Desinfektionsmittel aus einem pflanzlichen Öl oder einer Mischung aus pflanzlichen Ölen besteht.

Der Begriff "pflanzliche Öle", wie er hier verwendet wird, bezieht sich immer auf pflanzliche Öle, die desinfizierende Eigenschaften, insbesondere antimikrobielle und/oder antibakterielle und/oder mycostatische und/oder bakteriostatische Wirkung haben.

Pflanzliche Öle sind deshalb bevorzugt, weil sie im allgemeinen relativ ungiftig und gut verträglich sind und durch ihre ölige, d.h. hydrophobe Natur nicht oder nur wenig ausgewaschen werden. Dadurch wird deren Verbleib im Scheidenbereich auch bei längerem Aufenthalt im Wasser gewährleistet und deren Abgabe an die Umwelt minim gehalten. Zudem sind entsprechend behandelte Tampons problemlos entsorgbar.

Grundsätzlich kann das Desinfektionsmittel neben dem pflanzlichen Öl auch weitere wirksame Substanzen enthalten, insbesondere für den gezielten Einsatz gegen einen speziellen Infektionserreger. Ein solcher Zusatz ist aber üblicherweise zu vermeiden, da viele solche Zusätze ausgewaschen werden, was deren Wirkung beeinträchtigt und zu einer unerwünschten Umweltbelastung führen kann.

### Wege zur Ausführung der Erfindung

Das im erfindungsgemässen Tampon eingesetzte Imprägniermittel umfasst vorzugsweise als Desinfektionsmittel ein pflanzliches Öl oder eine Mischung pflanzlicher Öle.

Pflanzliche Öle oder Mischungen pflanzlicher Öle, die desinfizierende Eigenschaften haben sind bekannt.

Gegenwärtig bevorzugte pflanzliche Öle sind Teebaumöl und Johannisöl (auch bekannt als Johanniskrautöl) oder eine Kombination der beiden. Diese Öle entfalten sowohl antimikrobielle als auch antibakterielle, mycostatische und bakteriostatische Wirkung. Ferner hat sich gezeigt, dass diese Öle auch bei längerem Aufenthalt im Wasser kaum ausgewaschen werden.

Der erfindungsgemässe Tampon besitzt gewöhnlich ein Imprägniermittel mit einem Träger, der halbfest oder fest ist. Dieses Kriterium macht einerseits die Handhabung einfacher, da die Hände sauber bleiben, andererseits eignen sich solche Träger sehr gut für die Fixierung des als Desinfektionsmittel verwendeten Öls. Der Träger weist vorzugsweise einen Schmelzpunkt von etwa 38°C bis etwa 65°C auf. Speziell geeignet als ein solcher Träger ist ein Paraffin oder eine Mischung von Paraffinen, insbesondere weisses Petrolatum (Vaseline) mit einem Schmelzpunkt von ca. 38 - 60°C, einem Siedepunkt von über 300°C und einer Dichte von 0.820 - 0.880.

Üblicherweise enthält das Imprägniermittel das Desinfektionsmittel in einer Menge von 1 bis 10 Gew.-% bezogen auf das Gewicht des Trägers. Für ein Imprägniermittel, das als Desinfektionsmittel Johannisöl enthält ist eine Menge von mindestens etwa 2 Gew.-%, üblicherweise etwa 5 Gew.-% des Johannisöls bevorzugt, für eines, das Teebaumöl enthält eine Menge von mindestens etwa 4 Gew.-%, üblicherweise 4 bis 5 Gew.-% Teebaumöl, beide bezogen auf das Gewicht des Trägers, insbesondere Vaselin.

Obschon ein erfindungsgemässer Tampon grundsätzlich als Badeschutz eingesetzt werden kann ist es bevorzugt, wenn er zugleich im Zeitpunkt der Monatsblutung einsetzbar ist. Zu diesem Zweck muss er nicht nur den Eintritt von Wasser in die Scheide verhindern sondern gleichzeitig auch Blut aufsaugen. Diese Anforderungskombination wird durch einen Tampon erfüllt, der nur teilimprägniert ist, und zwar derart, dass er bei Gebrauch wasserseitig, d.h. bei üblicher Tamponausführung auf der Seite des Austritts des Haltemittels für dessen Entfernung aus dem saugfähigen Tampon-Körper, eine vollständig imprägnierte Oberfläche aufweist, der dem Austritt des Haltemittels entgegengesetzte Teil aber zumindest teilweise frei von Imprägniermittel ist. Dies kann auf unterschiedliche Arten erreicht werden, z.B. durch nur teilweises Eintauchen in das Imprägniermittel, durch Abschirmung bestimmter Bereiche, z.B. durch Eintauchen oder Besprühen in einer Art Siebhalterung etc. Ein sehr einfaches Verfahren besteht darin, den Tampon mit der Austrittseite des Haltemittels, üblicherweise einer Schnur, voran in das Imprägniermittel einzutauchen. Durch dieses Tauchverfahren wird zudem auch mindestens ein dem Saugkörper benachbarter Teil des Haltemittels imprägniert, was für die infektionshemmende Wirkung positiv ist.

Die nicht imprägnierte Stelle macht vorzugsweise zwischen 10 und 20 %, insbesondere zwischen 12 - 15 % der Tamponoberfläche aus.

Ein Tampon-Körper kann ein- oder mehrteilig konzipiert sein. Für einen guten Badeschutz wesentlich ist aber, dass der Tampon-Körper als Ganzes mindestens haltemittelseitig mit Imprägniermittel versehen ist, da beim Imprägnieren von z.B. nur einer äusseren Hülle, die nachträglich auf einen "Kern" aufgebracht wird, die Hülle um das Haltemittel nicht dicht geschlossen werden kann, so dass zumindest im Bereich des Haltemittels Wasser in den Tampon eindringen und damit sowohl dessen Saugwirkung als auch den Infektionsschutz vermindern könnte. Eine bevorzugte Ausführungsform des erfindungsgemässen Tampons umfasst den als solchen mindestens haltemittelseitig imprägnierten Tampon-Körper und das ev. ebenfalls zumindest teilimprägnierte Haltemittel.

Ein einfaches Verfahren zur Herstellung eines erfindungsgemässen Tampons zeichnet sich dadurch aus, dass das imprägnierende Mittel auf eine Temperatur gebracht wird, die mindestens 10°C über dem Schmelzpunkt des Trägers liegt, dass der Tampon mindestens teilweise in das imprägnierende Mittel eingetaucht wird und dass der Tampon anschliessend durch abtropfen lassen von überschüssigem Imprägniermittel befreit und auf Raumtemperatur abgekühlt wird. Eine bevorzugte Temperatur für das Aufbringen eines Imprägniermittels mit der bevorzugten weissen Vaseline als Träger ist ca. 90°C - 92°C. Höhere Temperaturen sollten aus Rücksicht auf die pflanzlichen Öle vermieden werden. Das Imprägniermittel kann entweder zuvor gemischt werden oder aber direkt vor dem Imprägnierschritt. Wesentlich ist lediglich eine homogene Durchmischung zum Zeitpunkt des Impränierschrittes. Ein zu langes Halten des Imprägniermittels auf hoher Temperatur sollte vermieden werden. Bei einer Temperatur des Imprägniermittels von ca. 92°C hat sich eine Eintauchzeit von 1 bis 2 Minuten bewährt. Anschliessend an das Eintauchen folgt eine Abtropfphase von vorzugsweise mindestens ca. 20 Sekunden, worauf der Tampon auf Raumtemperatur abgekühlt wird. Diese Abkühlung kann entweder durch Umgebungsluft oder - beschleunigt - z.B. durch Zufuhr kühler Luft erfolgen.

## Patentansprüche

1. Mit Imprägniermittel versehener Tampon mit einem saugfähigen Tampon-Körper und einem einseitig aus dem Tampon-Körper austretenden Haltemittel zur Entfernung des Tampons aus der Scheide, **dadurch gekennzeichnet, dass** das Imprägniermittel einen physiologisch unbedenklichen hydrophoben Träger und ein im wesentlichen ebenfalls hydrophobes und physiologisch unbedenkliches Desinfektionsmittel enthält und dass der Tampon-Körper zumindest auf der Seite des Austritts des Haltemittels aus dem Tampon-Körper eine vollständig imprägnierte Oberfläche aufweist.

2. Tampon gemäss Anspruch 1, **dadurch gekennzeichnet, dass** das hydrophobe Desinfektionsmittel mindestens eine der folgenden Wirkungen entfaltet:
antimikrobielle Wirkung, antibakterielle Wirkung, mycostatische Wirkung und bakteriostatische Wirkung.

3. Tampon gemäss Anspruch 1 oder 2, **dadurch gekennzeichnet, dass** das hydrophobe Desinfektionsmittel ein pflanzliches Öl oder eine Mischung von pflanzlichen Ölen enthält.

4. Tampon gemäss Anspruch 3, **dadurch gekennzeichnet, dass** das hydrophobe Desinfektionsmittel aus einem pflanzlichen Öl oder eine Mischung von pflanzlichen Ölen besteht.

5. Tampon gemäss Anspruch 3 oder 4, **dadurch gekennzeichnet, dass** das pflanzliche Öl Teebaumöl und/oder Johanniskrautöl ist.

6. Tampon gemäss einem der Ansprüche 1 bis 5, **dadurch gekennzeichnet, dass** der Träger hälbfest oder fest ist.

7. Tampon gemäss einem der Ansprüche 1 bis 6, **dadurch gekennzeichnet, dass** der Träger einen Schmelzpunkt von etwa 38°C bis etwa 65°C aufweist.

8. Tampon gemäss einem der Ansprüche 1 bis 7, **dadurch gekennzeichnet, dass** der Träger ein Paraffin oder eine Mischung von Paraffinen, insbesondere weisses Petrolatum (Vaseline) ist.

9. Tampon gemäss einem der Ansprüche 1 bis 8, **dadurch gekennzeichnet, dass** das Imprägniermittel das Desinfektionsmittel in einer Menge von 1 bis 10 Gew.-% bezogen auf das Gewicht des Trägers enthält.

10. Tampon gemäss einem der Ansprüche 1 bis 9, **dadurch gekennzeichnet, dass** das Imprägniermittel als Desinfektionsmittel entweder Johanniskrautöl in einer Menge von 2 Gew.-% oder Teebaumöl in einer Menge von 4 Gew.-% bezogen auf das Gewicht des Trägers enthält.

11. Tampon gemäss einem der Ansprüche 1 bis 9, **dadurch gekennzeichnet, dass** das Imprägniermittel als Desinfektionsmittel entweder Johannikrautöl oder Teebaumöl in einer Menge von 5 Gew.-% bezogen auf das Gewicht des Trägers enthält.

12. Tampon gemäss einem der Ansprüche 1 bis 11, **dadurch gekennzeichnet, dass** er nur teilimprägniert ist, wobei die dem Austritt des Haltemittels entgegengesetzte Seite eine Oberfläche aufweist, die zumindest teilweise frei von Imprägniermittel ist.

13. Tampon gemäss einem der Ansprüche 1 bis 12, **dadurch gekennzeichnet, dass** er auf der dem Austritt des Haltemittels entgegengesetzten Seite eine nicht imprägnierte Stelle aufweist, die zwischen 10 und 20 %, insbesondere 12 - 15 % der Tamponoberfläche ausmacht.

14. Verfahren zur Herstellung eines Tampons gemäss einem der Ansprüche 1 bis 13, **dadurch gekennzeichnet, dass** das Imprägniermittel auf eine Temperatur gebracht wird, die mindestens 10°C über dem Schmelzpunkt des Trägers liegt, vorzugsweise aber 92°C nicht überschreitet, dass der Tampon mindestens teilweise während einer angemessenen Imprägnierzeit in das imprägnierende Mittel eingetaucht wird und dass der Tampon anschliessend durch Abtropfenlassen während einer angemessenen Abtropfzeit von überschüssigem Imprägniermittel befreit und auf Raumtemperatur abgekühlt wird.

15. Verfahren gemäss Anspruch 14, **dadurch gekennzeichnet, dass** die Temperatur des Imprägniermittels 92°C beträgt, die Eintauchzeit 1 bis 2 Minuten und die Abtropfzeit 20 Sekunden.

16. Tampons gemäss einem der Ansprüche 1 bis 13 als Badeschutz.

## Claims

1. Tampon provided with impregnating agent having an absorbent tampon body and a holding means extending from said tampon body on one side for removal of said tampon from the vagina, **characterized in that** the impregnating agent comprises a physiologically harmless hydrophobic carrier and an essentially also hydrophobic and physiologically harmless desinfectant and that the tampon body has a completely impregnated surface at least on the side of the exit of said holding means.

2. Tampon according to claim 1, **characterized in that** the hydrophobic desinfectant exhibits at least one of the following effects:
antimicrobial effect, antibacterial effect, mycostatic effect and bacteriostatic effect.

3. Tampon according to claim 1 or 2, **characterized in that** the hydrophobic desinfectant contains a plant oil or a mixture of plant oils.

4. Tampon according to claim 3, **characterized in that** the hydrophobic desinfectant consists of a plant oil or a mixture of plant oils.

5. Tampon according to claim 3 or 4, **characterized in that** the plant oil is tee tree oil and/or hypericon oil.

6. Tampon according to anyone of claims 1 to 5, **characterized in that** the carrier is semi-solid or solid.

7. Tampon according to anyone of claims 1 to 6, **characterized in that** the carrier has a melting point of about 38°C to about 65°C.

8. Tampon according to anyone of claims 1 to 7, **characterized in that** the carrier is a paraffin or a mixture of paraffins, in particular white petrolatum (vaseline).

9. Tampon according to anyone of claims 1 to 8, **characterized in that** the impregnating agent comprises the desinfectant in an amount of from 1 to 10 % by weight referred to the weight of carrier.

10. Tampon according to anyone of claims 1 to 9, **characterized in that** the impregnating agent comprises as desinfectant either hypericon oil in an amount of 2 % by weight or tee tree oil in an amount of 4 % by weight referred to the weight of the carrier.

11. Tampon according to anyone of claims 1 to 9, **characterized in that** the impregnating agent comprises as desinfectant either hypericon oil or tee tree oil in an amount of 5 % by weight referred to the weight of the carrier.

12. Tampon according to anyone of claims 1 to 11, **characterized in that** it is only partially impregnated, whereby the side opposite to the exit of the holding means has a surface that is at least partially free of impregnating agent.

13. Tampon according to anyone of claims 1 to 12, **characterized in that** at the side opposite to the exit of the holding means it has a not impregnated part corresponding to between 10 and 20 %, in particular 12 to 15 % of the whole tampon surface.

14. Method for the production of a tampon according to anyone of claims 1 to 13, **characterized in that** the impregnating agent is heated to a temperature that is at least 10°C above the melting point of the carrier, preferably, however, does not exceed 92°C, that for an adequate impregnation time the tampon is at least partially immersed into said impregnating agent, and that then excess impregnating agent is removed from the tampon by letting it drop off during an adequate dropping off time, and cooling to room temperature.

15. Method according to claim 14, **characterized in that** the temperature of the impregnating agent is 92°C, the immersion time is 1 to 2 minutes and the dropping off time 20 seconds.

16. Tampon according to anyone of claims 1 to 13 as protection during taking a bath.

## Revendications

1. Tampon pourvu d'un agent d'imprégnation avec un corps de tampon absorbant et un moyen de saisie sortant d'un côté du corps de tampon pour l'enlèvement du tampon hors du vagin, **caractérisé en ce que** l'agent d'imprégnation contient un porteur hydrophobe ne présentant aucun risque physiologique et un agent désinfectant, essentiellement également hydrophobe et ne présentant aucun risque physiologique, et **en ce que** le corps de tampon présente une surface entièrement imprégnée au moins sur le côté de la sortie du moyen de saisie hors du corps de tampon.

2. Tampon selon la revendication 1, **caractérisé en ce que** l'agent désinfectant hydrophobe exerce au moins une des actions suivantes:
action antimicrobienne, action antibactérienne, action mycostatique et action bactériostatique.

3. Tampon selon la revendication 1 ou 2, **caractérisé en ce que** l'agent désinfectant hydrophobe contient une huile végétale ou un mélange d'huiles végétales.

4. Tampon selon la revendication 3, **caractérisé en ce que** l'agent désinfectant hydrophobe se compose d'une huile végétale ou d'un mélange d'huiles végétales.

5. Tampon selon la revendication 3 ou 4, **caractérisé en ce que** l'huile végétale est l'huile de théier et/ou l'huile de millepertuis.

6. Tampon selon l'une quelconque des revendications 1 à 5, **caractérisé en ce que** le porteur est semi-solide ou solide.

7. Tampon selon l'une quelconque des revendications 1 à 6, **caractérisé en ce que** le porteur présente un point de fusion d'environ 38°C à environ 65°C.

8. Tampon selon l'une quelconque des revendications 1 à 7, **caractérisé en ce que** le porteur est une paraffine ou un mélange de paraffines, en particulier du pétrolatum blanc (vaseline).

9. Tampon selon l'une quelconque des revendications 1 à 8, **caractérisé en ce que** l'agent d'imprégnation contient l'agent désinfectant en une quantité de 1 à 10 % en poids rapportée au poids du porteur.

10. Tampon selon l'une quelconque des revendications 1 à 9, **caractérisé en ce que** l'agent d'imprégnation contient comme agent désinfectant soit de l'huile de millepertuis en une quantité de 2 % en poids soit de l'huile de théier en une quantité de 4 % en poids rapportée au poids du porteur.

11. Tampon selon l'une quelconque des revendications 1 à 9, **caractérisé en ce que** l'agent d'imprégnation contient comme agent désinfectant soit de l'huile de millepertuis soit de l'huile de théier en une quantité de 5 % en poids rapportée au poids du porteur.

12. Tampon selon l'une quelconque des revendications 1 à 11, **caractérisé en ce qu'**il n'est que partiellement imprégné, le côté opposé à la sortie du moyen de saisie présentant une surface qui est au moins en partie exempte d'agent d'imprégnation.

13. Tampon selon l'une quelconque des revendications 1 à 12, **caractérisé en ce qu'**il présente, sur le côté opposé à la sortie du moyen de saisie, une zone non imprégnée qui représente entre 10 et 20 %, en particulier 12 - 15 % de la surface du tampon.

14. Procédé pour la fabrication d'un tampon selon l'une quelconque des revendications 1 à 13, **caractérisé en ce que** l'agent d'imprégnation est porté à une température qui se situe au moins 10°C au-dessus du point de fusion du porteur, mais ne dépasse de préférence pas 92°C, **en ce que** le porteur est immergé au moins en partie dans l'agent d'imprégnation pendant une durée d'imprégnation appropriée, et **en ce que** le tampon est ensuite débarrassé de l'agent d'imprégnation en excès par égouttage pendant une durée d'égouttage appropriée et est refroidi à la température ambiante.

15. Procédé selon la revendication 14, **caractérisé en ce que** la température de l'agent d'imprégnation vaut 92°C, la durée d'immersion 1 à 2 minutes et la durée d'égouttage 20 secondes.

16. Tampon selon l'une quelconque des revendications 1 à 13 formant protection pour le bain.
